Europäisches Patentamt

European Patent Office

Office européen des brevets

(11) Publication number: **0 313 166**
**A1**

# EUROPEAN PATENT APPLICATION

(21) Application number: 88202332.8

(22) Date of filing: 19.10.88

(51) Int. Cl.⁴: **C07C 126/02**

(30) Priority: 23.10.87 NL 8702530

(43) Date of publication of application:
26.04.89 Bulletin 89/17

(84) Designated Contracting States:
AT BE CH DE ES FR GB IT LI NL SE

(71) Applicant: **STAMICARBON B.V.**
**Mijnweg 1**
**NL-6167 AC Geleen(NL)**

(72) Inventor: **Verweel, Cornelis**
**Boerhaavestraat 72**
**NL-6164 GT Geleen(NL)**

(54) Process for preparing urea and water starting from ammonia and carbon dioxide.

(57) Process for preparing urea, in which process - a urea and carbamate-containing urea synthesis solution is formed from $NH_3$ and $CO_2$,
- carbamate is decomposed by the supply of heat using high-pressure steam and the decomposition products are removed from the urea synthesis solution, while steam condensate is formed in the process,
- the resulting urea-containing solution is concentrated by evaporation, product urea is recovered and the vapour phase obtained in the process is condensed to form process condensate,
- the process condensate is treated for the decomposition of the urea present and for the removal of $NH_3$ and $CO_2$, while a flow of waste water is obtained containing traces of urea and $NH_3$, this process being characterized in that the flow of waste water is at least partly converted into contaminated low-pressure steam, this low-pressure steam is utilized in the urea process, with formation of contaminated condensate, this condensate is subjected to a flash treatment and the condensate remaining after the flash treatment is carried off and utilized as boiler feed water.

EP 0 313 166 A1

## PROCESS FOR PREPARING UREA AND WATER STARTING FROM AMMONIA AND CARBON DIOXIDE

The invention relates to the preparation of urea and water starting from ammonia and carbon dioxide. More particularly the invention relates to the preparation of boiler feed water from the reaction liquor formed in the urea preparation process.

In the preparation of urea from ammonia and carbon dioxide, a urea synthesis solution is formed at high temperature and at the pressure going with it, which solution still contains a substantial amount of free ammonia and non-converted ammonium carbamate. The carbamate is decomposed in one or more steps into ammonia and carbon dioxide, which are driven off for the greater part with the free ammonia present and usually recirculated. The heat required for the decomposition of the carbamate is mostly obtained by the condensation of steam of an adequate pressure and temperature level. The condensate formed can be used again as boiler feed water. In the final decomposition step an aqueous urea solution is obtained which still contains amounts of dissolved ammonia and carbon dioxide, which are subsequently removed by expansion to atmospheric or lower pressure. The aqueous urea solution is concentrated by evaporation and/or crystallization and processed further. In the evaporation and the crystallization a gas mixture is formed containing, in addition to water vapour, entrained fine drops of urea and further also ammonia and carbon dioxide. This gas mixture is condensed, as is the gas mixture separated off in the expansion of the urea solution after the final decomposition step. Thus a so-called process condensate is obtained, which is partly returned to the process for the purpose of absorbing the gas mixture carried off from the final decomposition step. The remaining part is usually discharged. The process condensate also incorporates the water introduced into the process as steam for the operation of the ejectors in the evaporation and/or crystallization section, washing water, rinsing water on the stuffing boxes of the carbamate pumps, etc. In the urea preparation process one mole water is formed per mole urea. So in a urea plant with a capacity of 1000 tonnes urea a day 300 tonnes water is formed every day. Besides, daily about 315 tonnes water is supplied, so that a total amount of about 615 tonnes water must be carried off every day.

Usually this water still contains 2-9% (wt) $NH_3$, 0.8-6% (wt) $CO_2$ and 0.3-1.5% (wt) urea. Before discharging the water, these not insubstantial amounts of raw materials and product are recovered, on the one side to restrict the loss of raw materials and product, on the other to prevent the surface water into which the waste water would have been discharged from being polluted by them to a degree no longer permitted by the governments in many countries.

A process frequently applied for the virtually complete removal of the amounts of ammonia and urea present in the process condensate before discharging the process condensate as waste water is known from European patent specification 53.410. According to the process described in that publication the hydrolysis of the urea in the process condensate previously liberated from part of the $NH_3$ and $CO_2$ by means of low-pressure steam is carried out countercurrently in a reaction column using high-pressure steam, the temperature maintained at the bottom of the column being 180-230ºC and at the top 170-220ºC. Owing to the hydrolizing and stripping effect of the high-pressure steam, it is possible to remove ammonia and urea from the flow of waste water down to a content of 10 ppm or less before discharging this flow. However, the presence of an amount of about 10 ppm urea in boiler feed water for high-pressure steam is not permitted, because the urea decomposes in the steam boiler into $NH_3$ and $CO_2$, and severe norms have been imposed in respect of the presence of $CO_2$ in boiler feed water. High-pressure steam is understood to mean steam having a pressure of at least 12 bar.

In the past few decades urea preparation facilities have been realized in territories in which the water required in the urea preparation process is not available in large amounts, or available in a condition requiring expensive processing to make the water suitable for use in steam boilers. This is the case, for instance, when boiler feed water must be prepared from seawater.

The object of the invention is to provide a process in which the flow of waste water to be discharged from the urea synthesis is made suitable for use as boiler feed water. It is noted that from the U.S. patent specification 3,922,222 it is known how to treat urea-containing process condensate to make it suitable as boiler feed water. According to the process described, the process condensate is evaporated completely at low pressure in a steam boiler or heat exchanger, the urea and biuret present in the condensate being decomposed into $NH_3$ and $CO_2$. The vapour formed is condensed and most of the $NH_3$ and $CO_2$ is stripped from the condensate. From the remaining liquid, which yet contains about 1500 ppm $NH_3$, most of the $NH_3$ is removed by heating with steam in a deaerator, upon which it can be used as boiler feed water.

The disadvantage of the process described is that the steam prepared from this boiler feed water is so-called contaminated steam, which is said by the publication to contain about 300 ppm $NH_3$. Such steam can be used only to a limited degree.

The invention now provides a process in which the process condensate obtained in the urea synthesis is converted into boiler feed water while avoiding the above-mentioned disadvantage. This is achieved according to the invention by converting the process condensate at least in part into contaminated low-pressure steam after the substantial removal of urea and $NH_3$ therefrom, only traces of these compounds being left in it, by utilizing this low-pressure steam in the urea process wherever possible and by the flash removal of any remaining $CO_2$ from the condensate formed from it.

The removal, to a high degree, of urea and $NH_3$ from the process condensate can be effected in any suitable manner, for instance in the manner described in European patent specification 53.410. In that process, however, it is not necessary per se to continue to remove the urea and $NH_3$ to the extremely low values of 6 ppm $NH_3$ and 8 ppm urea mentioned in that publication, but without objection a less rigorous removal to for instance 50-100 opm will suffice.

In flashing the condensate formed from the contaminated low-pressure steam, most of the $NH_3$ and $CO_2$ present in trace amounts is removed. It is important for particularly the amount of $CO_2$ to be reduced to a very low value, for instance to less than 1 ppm, in connection with the danger of corrosion in the steam boiler in which said condensate is converted into steam. The presence possibly of any ppm $NH_3$ is less objectionable for this purpose. The condensate remaining after flashing can be carried off and is suitable for the production of high-pressure steam. The invention therefore relates to a process for preparing urea in which
- a urea and carbamate-containing urea synthesis solution is formed from $NH_3$ and $CO_2$,
- carbamate is decomposed by the supply of heat using high-pressure steam and the decomposition products are removed from the urea synthesis solution, while steam condensate is being formed in the process,
- the resulting urea-containing solution is concentrated by evaporation, product urea is recovered and the vapour phase obtained in the process is condensed to form process condensate,
- the process condensate is treated for the decomposition of the urea present and for the removal of $NH_3$ and $CO_2$,
in which process a flow of waste water is obtained containing traces of urea and $NH_3$. The invention is characterized in that the flow of waste water is at least partly converted into contaminated low-pressure steam, this low-pressure steam is utilized in the urea process, with formation of contaminated condensate, this condensate is subjected to a flash treatment and the condensate remaining after the flash treatment is carried off and utilized as boiler feed water. As a rule the condensate remaining after the flash treatment contains less than 1 ppm $CO_2$ and is free of urea.

The condensate remaining after the flash treatment is preferably converted into high-pressure steam together with the steam condensate. At least a part of the contaminated low-pressure steam can be used for desorbing $NH_3$ and $CO_2$ from the process condensate in the preparation of the flow of waste water. A part of the contaminated low-pressure steam can be used also for the operation of the vacuum ejectors in the evaporation of the urea solution and/or be applied in indirect heat exchanges in the urea preparation process. If so desired, the condensate remaining after the flash treatment can be subjected, before its conversion into high-pressure steam, to a treatment using ion exchangers, so that any existing foreign compounds that can be bound to ion exchangers can be removed.

The big advantage of the process according to the invention is that most of the amount of water required in the urea synthesis is recovered from the waste water and from the steam condensate formed. Only a fraction of the amount required, usually less than 5%, must be supplied from elsewhere. Without applying the measures according to the invention it is only the steam condensate that will be available as boiler feed water and an amount of 40-50% will have to be supplied from elsewhere.

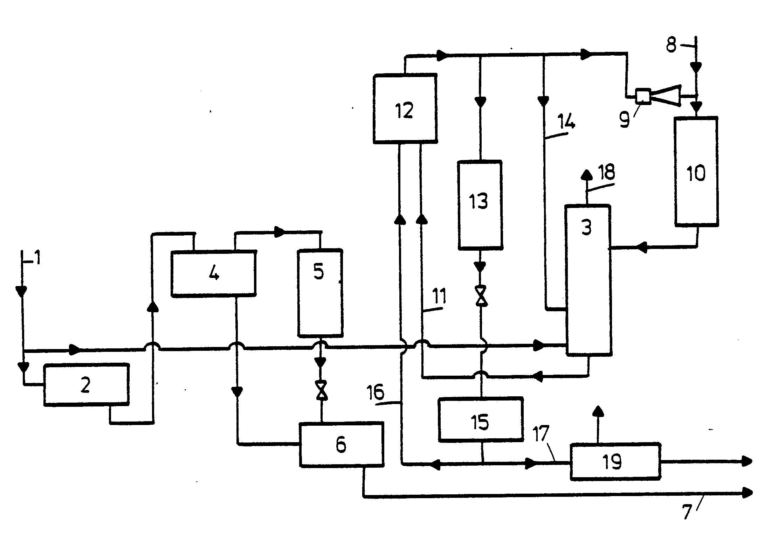

The invention will be elucidated by means of the figure and the example without, however, being limited thereto.

The lines shown represent steam and water flows in a urea plant operating according to the Stamicarbon $CO_2$ stripping process as described in, for instance, European Chemical News Urea Supplement pp. 17-20 of 17th January 1969.

Through 1 high-pressure steam with a pressure of, for instance, 20 bar is supplied to the urea plant, which high-pressure steam is used as a source of heat in a stripping device 2 and as hydrolyzing and stripping medium in the hydrolyzing facility 3 for hydrolyzing urea in the process condensate and driving out the formed $NH_3$ and $CO_2$ products of hydrolysis. The condensate obtained in 2 is expanded in a steam reservoir 4, which is kept under a pressure of, for instance, 9 bar, and converted in it into steam. The steam formed is used in equipment 5, for instance an evaporator heater for the concentration of the urea solution produced. Condensate is supplied to a steam condensate reservoir 6 and is subsequently carried off through 7 in order to be used again for the formation of steam. To the steam condensate reservoir 6 is supplied also the condensate formed in steam reservoir 4.

The water formed in the urea reaction is removed from the evaporating section in the form of impure water vapour, supplied through 8 to a condensing facility 10, together with the steam used for maintaining, by means of ejectors 9, the vacuum in the evaporating section, and condensed to form process condensate. The process condensate is treated in the manner known from European patent specification 53,410, in which treatment a flow of waste water containing less than 10 ppm urea and $NH_3$ and a vapour mixture containing $NH_3$, $CO_2$ and water vapour are carried off from hydrolyzing facility 3 through respectively 11 and 18. The flow of waste water 11 is supplied to the low-pressure steam reservoir 12, in which a pressure of, for instance, 4.5 bar is maintained. The $NH_3$-containing low-pressure steam formed in 12 can be used in the urea process itself, for instance as a source of heat (equipment 13), as a desorption agent through 14 for the removal of $NH_3$ and $CO_2$ from the process condensate prior to the treatment thereof in hydrolyzing facility 3 and for maintaining a vacuum in ejectors 9. The condensate formed in 13, which may yet contain $NH_3$, is collected in tank 15. If so desired, a part is recirculated to reservoir 12, the rest is carried off through 17 and expanded to a lower, for instance atmospheric, pressure. During the expansion any remaining $NH_3$ and $CO_2$ will escape, leaving boiler feed water free from urea and containing less than 1 ppm $CO_2$.

Example

a. For the operation of a urea plant with a capacity of 1000 tonnes a day 761 tonnes high-pressure steam is required. In the preparation of urea 300 tonnes water is formed every day. For the further processing of the process condensate and the operation of the ejectors 314 tonnes steam is required. Without applying the measures according to the invention an amount of steam condensate can be obtained of 761-314 = 447 tonnes. 300+314 = 614 tonnes waste water is formed.

b. For the operation of the same plant, while applying the measures according to the invention, 31 tonnes of water is evaporated in the expansion of the steam condensate to atmospheric pressure. The steam condensate now obtained is 761-31 = 730 tonnes. The waste water obtained, which can now be used a boiler feed water, amounts to 300+31 = 331 tonnes.

Claims

1. Process for preparing urea, in which process
- a urea and carbamate-containing urea synthesis solution is formed from $NH_3$ and $CO_2$,
- carbamate is decomposed by the supply of heat using high-pressure steam and the decomposition products are removed from the urea synthesis solution, while steam condensate is formed in the process,
- the resulting urea-containing solution is concentrated by evaporation, product urea is recovered and the vapour phase obtained in the process is condensed to form process condensate,
- the process condensate is treated for the decomposition of the urea present and for the removal of $NH_3$ and $CO_2$, while a flow of waste water is obtained containing traces of urea and $NH_3$, this process being characterized in that the flow of waste water is at least partly converted into contaminated low-pressure steam, this low-pressure steam is utilized in the urea process, with formation of contaminated condensate, this condensate is subjected to a flash treatment and the condensate remaining after the flash treatment is carried off and utilized as boiler feed water.

2. Process according to claim 1, characterized in that the condensate remaining after the flash treatment is converted into high-pressure steam together with the steam condensate.

3. Process according to claim 1 or 2, characterized in that at least a part of the contaminated low-pressure steam is used for the desorption of $NH_3$ and $CO_2$ from the process condensate in the preparation of the flow of waste water.

4. Process according to any one or more of claims 1-3, characterized in that a part of the contaminated low-pressure steam is used for the operation of the vacuum ejectors in the evaporation of the urea solution and/or in indirect heat exchanges in the urea preparation process.

5. Process according to any one or more of claims 1-4, characterized in that the condensate remaining after the flash treatment is subjected before its conversion into high-pressure steam to a treatment using ion exchangers.

6. Process for preparing urea and making boiler feed water as described and elucidated.

7. Boiler feed water obtained by applying the process according to any one or more of claims 1-5.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 053 410 (UK BU) * Whole document * | 1-7 | C 07 C 126/02 |
| D,A | US-A-3 922 222 (W.H. VAN MOORSEL) * Whole document * | 1-7 | |
| D,A | EUROPEAN CHEMICAL NEWS UREA SUPPLEMENT, 17th January 1969, pages 17-20; "DSM carbon dioxide stripping process" | 1-7 | |
| A | CHEMICAL ENGINEERING PROGRESS, vol. 73, no. 5, May 1977, pages 80-84, Aiche, New York, US; D.F. BRESS et al.: "The startup of two major urea plants" | 1-7 | |

TECHNICAL FIELDS
SEARCHED (Int. Cl.4)

C 07 C 126/00

The present search report has been drawn up for all claims

| Place of search | Date of completion of the search | Examiner |
|---|---|---|
| THE HAGUE | 24-11-1988 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                               

& : member of the same patent family, corresponding document

EPO FORM 1503 03.82 (P0401)